## Europäisches Patentamt

## European Patent Office

## Office européen des brevets

(19)

(11) Veröffentlichungsnummer: **0 071 699**
**B1**

(12) **EUROPÄISCHE PATENTSCHRIFT**

(45) Veröffentlichungstag der Patentschrift:
**19.02.86**

(51) Int. Cl.⁴: **A 61 F 5/44**

(21) Anmeldenummer: **82103396.6**

(22) Anmeldetag: **22.04.82**

(54) Sekretsammelvorrichtung.

(30) Priorität: **07.08.81 DE 3131378**

(43) Veröffentlichungstag der Anmeldung:
**16.02.83 Patentblatt 83/7**

(45) Bekanntmachung des Hinweises auf die Patenterteilung:
**19.02.86 Patentblatt 86/8**

(84) Benannte Vertragsstaaten:
**AT BE CH DE FR GB IT LI LU NL SE**

(56) Entgegenhaltungen:
**DE - A - 2 900 806**
**FR - A - 2 156 779**
**FR - A - 2 358 608**
**US - A - 3 312 221**
**US - A - 3 716 055**
**US - A - 4 192 295**

(73) Patentinhaber: **INTERMEDICAT GMBH,**
**Gerliswilstrasse 43, CH-6020 Emmenbrücke (CH)**

(72) Erfinder: **Voges, Karl-Friedrich, Ing. grad.,**
**Lindenbergstrasse 18, D-3508 Melsungen (DE)**

(74) Vertreter: **von Kreisler, Alek, Dipl.-Chem. et al,**
**Deichmannhaus am Hauptbahnhof, D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Sekretsammelvorrichtung mit einem Halter und einem an dem Halter aufzuhängenden verformbaren Behälter, der eine Zulaufleitung aufweist, welche mit dem Ende eines an dem Halter angeordneten Kanals abdichtend verbindbar ist.

Bei einer bekannten Sekretsammelvorrichtung (DE-A-2 900 806) ist ein flexibler Beutel vorgesehen, der mit Ösen an den Haken eines starren Halters befestigt wird. Der Halter weist einen nach unten gerichteten starren Kanal auf, an dem das Einlassende eines in den Sekretbeutel hineinführenden und mit dem Sekretbeutel fest verbundenen Schlauches befestigt wird. Zu diesem Zweck sind die Endstücke des Kanals und des Schlauches als ineinandergreifende Konusteile ausgebildet, die mit Druck ineinandergeschoben werden. Derartige Leitungskupplungen mit konischen Anschlussteilen haben jedoch den Nachteil, dass die Dichtigkeit der Verbindungen nur dann gewährleistet ist, wenn die beiden miteinander verbundenen Leitungsteile nicht in Längsrichtung auseinandergezogen werden. Eine geringfügige Längsverschiebung eines der konischen Verbindungsteile führt bereits zur Undichtigkeit. Hierdurch besteht die Gefahr, dass Körpersekret an der Verbindungsstelle austritt oder das Luft in den Beutel hinein gelangt. Werden die Verbindungselemente anschliessend wieder ineinandergedrückt, können sich in dem Beutel Lufteinschlüsse bilden, durch die nachfolgend das Einfliessen von Sekret in den Beutel erschwert wird.

Ferner ist eine Urinsammelvorrichtung bekannt, bei der in dem Sammelbeutel ein Folienventil angeordnet ist, das mit einem Nippel aus dem Sammelbeutel herausragt. Auf den Nippel ist der vom Patienten kommende Schlauch unter Aufweitung des Schlauchendes klemmend und festsitzend aufgeschoben (US-A-3 312 221).

Bei Sekretsammelvorrichtungen besteht der Behälter normalerweise aus einem flexiblen Beutel, dessen Folienwände bei zunehmender Füllung nach unten gezogen werden. Bei fester Ankopplung der mit dem Beutel verbundenen Zulaufleitung an den Kanal wird die Zulaufleitung einer Zugspannung ausgesetzt, die sich auf die Kopplungsvorrichtung überträgt.

Der Erfindung liegt die Aufgabe zugrunde, eine Sekretsammelvorrichtung der eingangs genannten Art zu schaffen, die Verschiebungen der Zulaufleitung relativ zu dem Kanal zulässt, ohne undicht zu werden und ohne dass grössere Materialspannungen entstehen.

Zur Lösung dieser Aufgabe ist erfindungsgemäss vorgesehen, dass die Zulaufleitung und der Kanal in ihren Verbindungsbereichen relativ zueinander unter Beibehaltung der Abdichtung selbsttätig teleskopartig längsverschiebbar sind und dass die Aufschiebelänge der Zulaufleitung auf dem Kanal so gross ist, dass bei der durch das Füllen des Behälters verursachten Verformung desselben die Abdichtung erhalten bleibt.

Bei der erfindungsgemässen Sekretsammelvorrichtung kann der Schlauch sich relativ zu dem Kanal in relativ weiten Grenzen verschieben, ohne dass Undichtigkeiten auftreten. Durch die teleskopartige Verbindung zwischen der Zulaufleitung und dem Kanal passt sich die Länge der Verbindungsvorrichtung selbsttätig den jeweiligen Verhältnissen an, insbesondere auch dann, wenn sich nach der Anbringung des Behälters an dem Halter die Lage oder Form des Behälters verändert. Als Sekretaufnahmebehälter wird in der Regel ein Beutel aus flexiblem Folienmaterial benutzt.

In vorteilhafter Ausgestaltung der Erfindung ist die Zulaufleitung ein flexibler Schlauch, der den als starres Rohr ausgebildeten Kanal umgreift. Der flexible Schlauch passt sich allen Lage- und Formänderungen des Beutels relativ zu dem Halter an. Da der Kanal als starres Rohr ausgebildet ist, ist die Kopplungsstelle in bezug auf den Halter unveränderbar festgelegt. Wichtig ist, dass die Länge, über die der Schlauch auf das Rohr aufgeschoben ist, hinreichend gross ist, um in dem erforderlichen Mass eine Längsverschiebung des Schlauches bzw. Verformung des Beutels zuzulassen.

In vorteilhafter Ausgestaltung der Erfindung weist das Rohr mindestens eine ringförmig umlaufende Verdickung in dem von dem Schlauch überdeckten Bereich auf. Hierdurch wird sowohl eine hinreichende Abdichtung der Konnektionsstelle gewährleistet und andererseits erreicht, dass beim Aufschieben des Schlauches auf das Rohr die Reibung weitgehend unabhängig von dem Mass des Aufschiebens ist, da diese Reibung im wesentlichen von der Verdickung bestimmt wird.

Eine weitere zweckmässige Ausgestaltung der Erfindung sieht vor, dass derjenige Bereich des Rohres, an dem der Schlauch befestigt ist, von einem das Rohrende überragenden Mantel mit radialem Abstand umgeben ist. Der Mantel bewirkt einen mechanischen Schutz der Konnektionsstelle, so dass der Schlauch nicht unbeabsichtigt von dem Rohr abgeschoben oder abgetrennt werden kann. Ferner wird durch den Mantel der Kontaminationsschutz verbessert.

Zweckmässigerweise sind das Rohr und der Mantel dem Halter einstückig angeformt, so dass separate Anschlusselemente überhaupt nicht benötigt werden.

Im folgenden werden unter Bezugnahme auf die Zeichnungen zwei Ausführungsbeispiele der Erfindung näher erläutert.

Es zeigen:

Figur 1 eine schematische Darstellung eines an einem Halter aufgehängten Urinsammelbeutels,
Figuren 2a bis 2d verschiedene Ausführungsformen des an dem Halter vorgesehenen Rohres,
Figur 3 einen Schnitt entlang der Linie III-III der Figur 1,
Figur 4 einen Schnitt entlang der Linie IV-IV der Figur 1,
Figur 5 in ähnlicher Darstellung wie Figur 1 eine zweite Ausführungsform der Sekretsammelvorrichtung,

Figur 6 einen Schnitt entlang der Linie VI-VI der Figur 5 und

Figur 7 einen Schnitt entlang der Linie VII-VII der Figur 5.

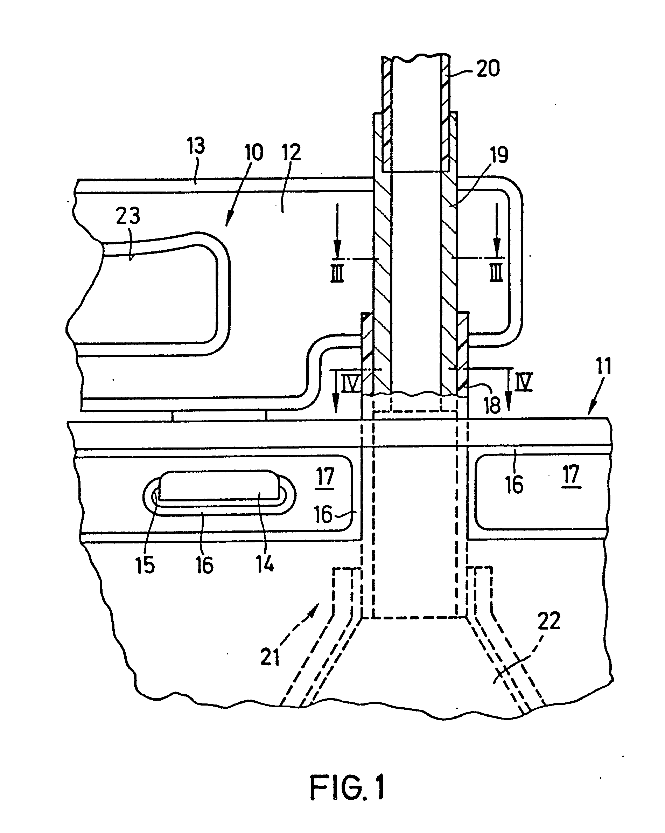

Die in Figur 1 dargestellte Sekretsammelvorrichtung besteht aus einem plattenförmigen Halter 10 und einem an dem Halter 10 zu befestigenden Beutel 11 aus flexiblem Folienmaterial. Der Halter 10 weist eine Platte 12 mit einem umlaufenden wulstförmigen Rand 13 auf. Von der Unterkante der Platte 12 stehen nach unten hin Laschen 14 ab, deren untere Enden hakenförmig umgebogen sind, so dass auf sie Ösen 15 des Beutels 11 aufgeschoben werden können, wodurch der Beutel 11 an dem Halter 10 aufgehängt wird. Der Beutel 11 besteht aus Folienteilen, die entlang ihrer oberen Ränder durch Siegelnähte 16 miteinander verbunden sind. Die Siegelnähte 16 verlaufen entlang des oberen Randes des Beutels 11 parallel, so dass sie langgestreckte Felder 17 umschliessen, in denen sich die ebenfalls von Siegelnähten 16 begrenzten Ösen 15 befinden.

Der Beutel 11 ist mit einem in das Beutelinnere hineinragenden vertikalen Schlauch 18 fest verbunden, der abdichtend zwischen den beiden Beutelfolien hindurchgeführt ist und sich über den oberen Beutelrand hinaus nach oben erstreckt.

Der Halter 10 weist ein vertikales zylindrisches Rohr 19 auf, das ihm einstückig angeformt ist, und an dessem oberen Ende das patientenferne Ende eines Sekretableitungsschlauches 20 befestigt ist. Das untere Ende des starren Rohres 19 ist mit einem Abstand vor der Platte 12 und dem Wulst 13 angeordnet und steht daher frei nach unten ab, d.h. seine kreisrunde Aussenfläche kommt mit keinem anderen Teil des Halters 10 in Kontakt. Über das untere Ende des Rohres 19 ist das obere Ende des zylindrischen Schlauches 18 geschoben, so dass auf dem Überlagerungsabschnitt der Schlauch 18 das Rohr 19 teleskopartig umgibt. Der Innendurchmesser des Schlauches 18 ist auf den Aussendurchmesser des Rohres 19 so abgestimmt, dass der Innenraum von Rohr 19 und Schlauch 18 gegen die Aussenluft abgedichtet ist. Dennoch kann der Schlauch 18 sich auf dem Rohr 19 in Längsrichtung verschieben.

Der Schlauch 18 steht im Innern des Hohlraumes 21 des Beutels 11 ein Stück über die untere Siegelnaht 16 hinaus vor. An dem unteren Ende des Schlauches 18 ist ein Folienventil 22 befestigt, dessen oberer Rand um das untere Schlauchende herumgelegt ist und das zwei flach aneinanderliegende Folienteile aufweist, zwischen denen das aus dem Schlauch 18 herabfallende Sekret heruntersinkt. Das Folienventil 22 verhindert andererseits das Aufsteigen von in dem Hohlraum 21 befindlicher Flüssigkeit in den Schlauch 18 hinein.

An dem Halter 10, an dem Beutel 11 oder im Zuge des Sekretableitungsschlauches 20 kann in bekannter Weise ein (nicht dargestelltes) Entlüftungsventil vorgesehen sein.

Der Halter 10 weist eine Greiföse 23 in Form eines Durchbruchs in der Platte 12 auf, so dass der Halter 10 zusammen mit dem an ihm angehängten Beutel 11 transportiert werden kann. Beim Gebrauch wird der Halter 10 an dem Bett des Patienten oder an einer Aufhängevorrichtung befestigt.

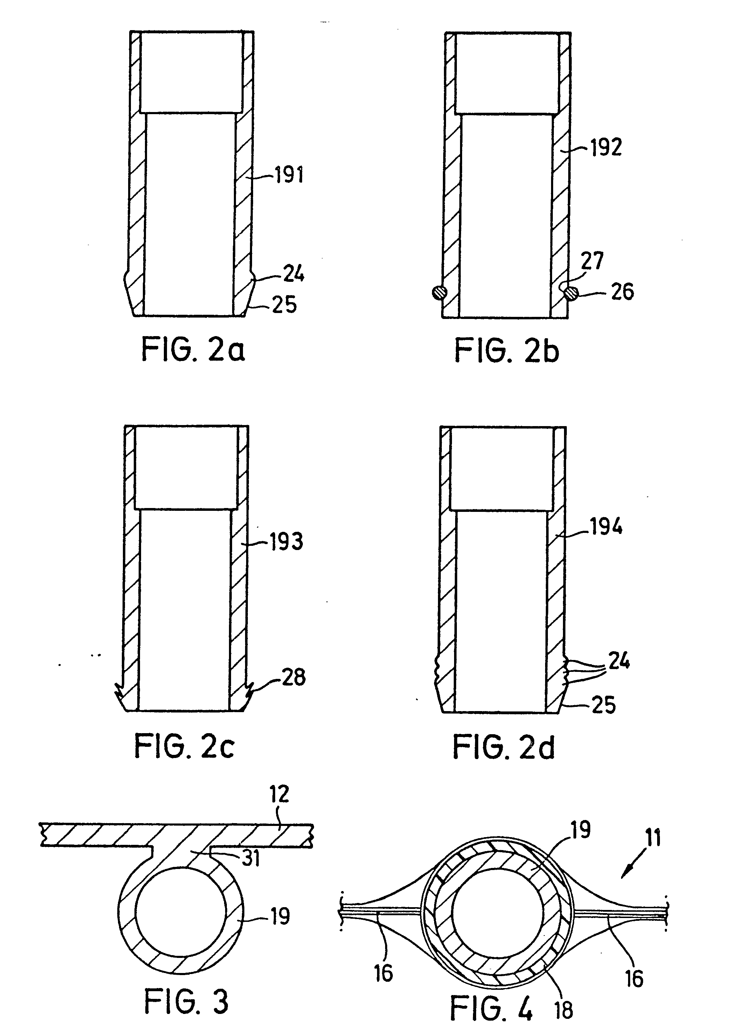

Während bei dem Ausführungsbeispiel der Figur 1 das Rohr 19 eine durchgehend zylindrische Aussenfläche hat, sind in den Figuren 2a bis 2d verschiedene Ausführungsformen des Rohres dargestellt, bei denen das Rohr im unteren Bereich eine umlaufende Verdickung aufweist, über die der Schlauch 18 geschoben wird und die im Zusammenwirken mit dem Schlauch 18 eine örtliche Dichtungsstelle bildet. Bei dem Ausführungsbeispiel der Figur 2a ist das Rohr 191 in der Nähe seines unteren Endes mit einem Umfangswulst 24 versehen, dessen untere Begrenzungskante 25 kegelförmig bis zum unteren Rand des Rohres 191 ausläuft, wobei die Wandstärke des Rohres 191 am unteren Rand kleiner ist als in dem oberhalb des Wulstes 24 liegenden Bereich. Auf diese Weise wird das Aufschieben des Schlauches 18 erleichtert.

Gemäss Figur 2b weist das Rohr 192 einen O-Ring 26 auf, der in eine umlaufende Nut 27 mit teilkreisförmigem Querschnitt am unteren Ende des Rohres 192 eingesetzt ist. Dieser O-Ring übernimmt nach dem Aufschieben des flexiblen Schlauches 18 die Hauptabdichtung, wobei der Schlauch 18 über dem O-Ring 26 radial aufgeweitet werden kann, so dass er auch über dem O-Ring 26 mit seiner Innenfläche an der Aussenfläche des Rohres 192 anliegt.

Figur 2c zeigt ein Rohr 193, das an seiner Umfangsfläche sägezahnförmige Zacken 28 aufweist. Diese Zacken 28 ermöglichen das Aufschieben des Schlauches 18 auf das Rohr 193, erschweren jedoch das Bewegen des Schlauches in Gegenrichtung, also nach unten. Bei dem Ausführungsbeispiel der Figur 2d sind mehrere Ringwülste 24 vorgesehen, die in dichter Folge hintereinander im unteren Endbereich des Rohres 194 angeordnet sind ,wobei der untere Ringwulst 24 in eine konische Fläche 25 übergeht, wie schon anhand von Figur 2a beschrieben worden ist.

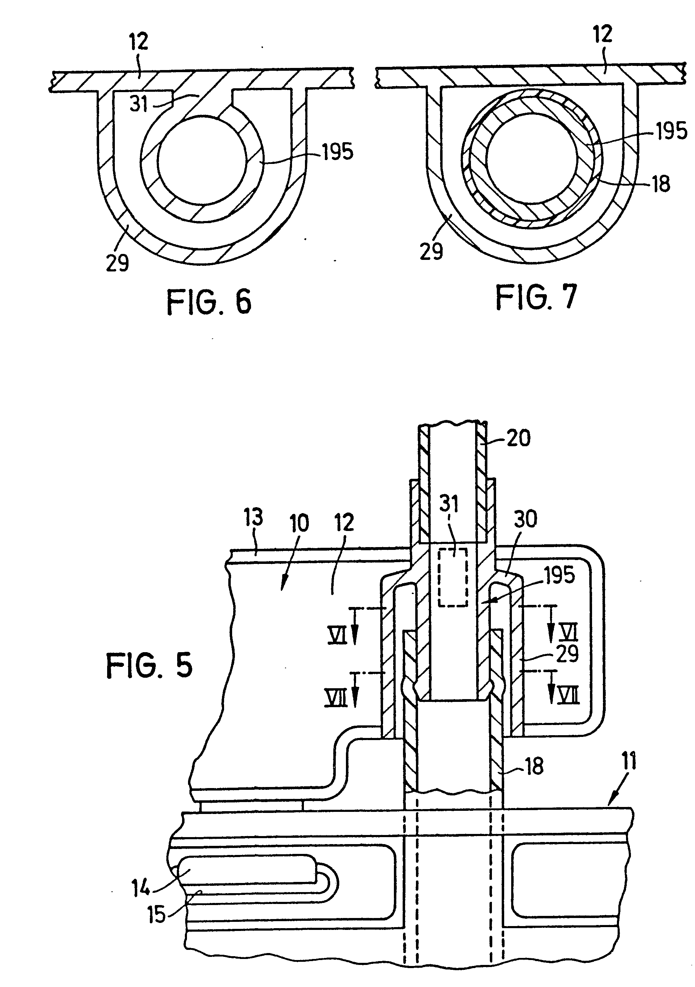

Bei dem Ausführungsbeispiel der Figur 5 wird der gleiche Beutel 11 benutzt wie bei dem Ausführungsbeispiel der Figur 1. Auch der Halter 10 entspricht im wesentlichen demjenigen der Figur 1. Das die beiden Ausführungsbeispiele unterscheidende Merkmal besteht darin, dass bei der Sekretsammelvorrichtung der Figur 5 das Rohr 195 auf einem Teil seiner Länge von einem Mantel 29 umgeben ist, der über das untere Ende des Rohres 195 hinaus vorsteht. Dieser Mantel 29 hat im wesentlichen U-förmigen Querschnitt, wobei die Enden seiner Schenkel senkrecht gegen die Platte 10 stossen und in diese übergehen. Die Innenseite des Mantels 29 hat einen Abstand von der Umfangsfläche des Rohres 195, so dass um das um den unteren Abschnitt des Rohres 195 herum ein nur nach unten hin offener Raum zum Einschieben des Schlauches 18 gebildet wird. Das obere Ende des Mantels 29 ist durch eine ringförmige Querwand 30 mit dem Rohr 95 verbunden.

Bei den vorstehend beschriebenen Ausführungsbeispielen ist jeweils das Rohr einstückig mit dem Halter 10 aus Kunststoff gefertigt. Die Verbindungsstelle, an der die Wand des Rohres mit dem Halter 10 verbunden ist, liegt jeweils oberhalb desjenigen Bereichs, in den der Schlauch 18 vorgeschoben wird. Diese Verbindungsstelle ist in den Figuren 3 und 5 mit 31 bezeichnet.

## Patentansprüche

1. Sekretsammelvorrichtung mit einem Halter (10) und einem an dem Halter aufzuhängenden verformbaren Behälter (11), der eine Zulaufleitung (Schlauch 18) aufweist, welche mit dem Ende eines an dem Halter angeordneten Kanals (Rohr 19) abdichtend verbindbar ist, dadurch gekennzeichnet, dass die Zulaufleitung (Schlauch 18) und der Kanal (Rohr 19) in ihren Verbindungsbereichen relativ zueinander unter Beibehaltung der Abdichtung selbsttätig teleskopartig längsverschiebbar sind und dass die Aufschiebelänge der Zulaufleitung auf dem Kanal so gross ist, dass bei der durch das Füllen des Behälters (11) verursachten Verformung desselben die Abdichtung erhalten bleibt.

2. Sekretsammelvorrichtung nach Anspruch 1, dadurch gekennzeichnet, dass die Zulaufleitung ein flexibler Schlauch (18) ist, der den als starres Rohr (19) ausgebildeten Kanal umgreift.

3. Sekretsammelvorrichtung nach Anspruch 1 oder 2, dadurch gekennzeichnet, dass das starre Rohr (19) an das patientenferne Ende eines Sekretableitungsschlauches (20) angeschlossen ist.

4. Sekretsammelvorrichtung nach einem der Ansprüche 1 bis 3, dadurch gekennzeichnet, dass das Rohr (191, 192, 193, 194) mindestens eine ringförmig umlaufende Verdickung (24, 26, 28) in dem von dem Schlauch (18) überdeckten Bereich aufweist.

5. Sekretsammelvorrichtung nach einem der vorhergehenden Ansprüche, dadurch gekennzeichnet, dass der Schlauch (18) sich im wesentlichen geradlinig von dem Beutel (11) über das Ende des Rohres (19) erstreckt.

6. Sekretsammelvorrichtung nach einem der Ansprüche 1 bis 5, dadurch gekennzeichnet, dass derjenige Bereich des Rohres (195), auf den der Schlauch (18) aufgeschoben ist, von einem das Rohrende überragenden Mantel (29) mit radialem Abstand umgeben ist.

7. Sekretsammelvorrichtung nach einem der vorhergehenden Ansprüche, dadurch gekennzeichnet, dass das Rohr (195) und ggf. der Mantel (29) dem Halter (10) einstückig angeformt ist.

## Claims

1. Secretion collecting device comprising a holder (10) and a deformable container (11) to be suspended at the holder, said container including a feed conduit (hose 18) which is sealingly connectible to the end of a channel (tube 19) provided at the holder, characterized in that the feed conduit (hose 18) and the channel (tube 19) may be automatically displaced telescopically relative to each other in their connecting areas while maintaining the sealing effect, and that the slip-on length of the feed conduit onto the channel is as large as to maintain the sealing in view of the deformation caused by the filling of the container (11).

2. Secretion collecting device according to claim 1, characterized in that the feed conduit is a flexible hose (18) encompassing the channel designed as a rigid tube (19).

3. Secretion collecting device according to claim 1 or 2, characterized in that the rigid tube (19) is connected to the distal end of a secretion discharge hose (20).

4. Secretion collecting device according to one of claims 1 to 3, characterized in that the tube (191, 192, 193, 194) contains at least one annular-type circumjacent enlargement (24, 26, 28) in the region covered by the hose (18).

5. Secretion collecting device according to one of the preceding claims, characterized in that the hose (18) extends substantially rectilinearly from the container (11) over the end of tube (19).

6. Secretion collecting device according to one of claims 1 to 5, characterized in that the region of the tube (195) on which the hose (18) is slipped is encompassed by a jacket (29) projecting beyond the tube end and being spaced radially from the tube.

7. Secretion collecting device according to one of the preceding claims, characterized in that the tube (195) and, optionally the jacket (29) are integrally formed with the holder (10).

## Revendications

1. Dispositif collecteur de secrétions comprenant un support (10) et un récipient déformable (11) accroché à ce support et comportant une conduite d'entrée (tuyau 18) susceptible d'être reliée de manière étanche à l'extrémité d'un canal (tube 19) disposé sur le support, caractérisé par le fait que la conduite d'entrée (tuyau 18) et le canal (tube 19) peuvent coulisser longitudinalement de manière télescopique et indépendamment l'un par rapport à l'autre dans leurs zones de jonction avec conservation de l'étanchéité, et que la longueur d'emmanchement de la conduite d'entrée sur le conduit est suffisamment grande pour que l'étanchéité subsiste lors de la déformation du récipient (11) provoquée par son remplissage.

2. Dispositif collecteur de sécrétions selon la revendication 1, caractérisé par le fait que la conduite d'entrée est un tuyau souple (18) qui enserre le conduit, ce dernier étant un tube rigide (19).

3. Dispositif collecteur de sécrétions selon l'une des revendications 1 et 2, caractérisé par le fait que le tube rigide (19) est relié à l'extrémité éloignée du patient d'un tuyau d'évacuation de sécrétions (20).

4. Dispositif collecteur de sécrétions selon l'une des revendications 1 à 3, caractérisé par le fait que le tube (191, 192, 193, 194) présente au moins un épaississement annulaire (24, 26, 28) dans la zone recouverte par le tuyau (18).

5. Dispositif collecteur de sécrétions selon l'une des revendications précédentes, caractérisé par le fait qu'à partir de la poche (11) le tuyau (18) s'étend sensiblement en ligne droite au-dessus de l'extrémité du tube (19).

6. Dispositif collecteur de sécrétions selon l'une des revendications 1 à 5, caractérisé par le fait que la zone (195) du tube sur laquelle est emmanché le tuyau (18) est entourée avec un espace radial d'une gaine (29) s'étendant au-delà de l'extrémité du tube.

7. Dispositif collecteur de sécrétions selon l'une des revendications précédentes, caractérisé par le fait que le tube (195) et, le cas échéant, la gaine (29) sont formés en une seule pièce avec le support (10).

FIG. 1

FIG. 2a

FIG. 2b

FIG. 2c

FIG. 2d

FIG. 3

FIG. 4

FIG. 6

FIG. 7

FIG. 5